# EUROPEAN PATENT SPECIFICATION

(11) **EP 1 420 818 B1**
(45) Date of publication and mention of the grant of the patent: **21.11.2018**
(21) Application number: 02760915.5
(22) Date of filing: 26.07.2002
(51) Int. Cl.: A61K 9/00, A61K 39/04, A61K 47/12

(54) **ANTIGENIC COMPOSITIONS**
ANTIGENE ZUSAMMENSETZUNGEN
COMPOSITIONS ANTIGENIQUES

(30) Priority: 26.07.2001 NZ 51316901
(43) Date of publication of application: 26.05.2004
(60) Divisional application: 10188891.5
(73) Proprietor: Otago Innovation Limited, Dunedin (NZ); Animal Health Board, Inc., Wellington (NZ); AgResearch Limited, Hamilton 2001 (NZ)
(72) Inventor: ALDWELL, Frank, Ernest, Dunedin (NZ); BUDDLE, Bryce, Malcolm, Upper Hutt (NZ); TUCKER, Ian, George, Dunedin (NZ)
(74) Representative: HGF Limited
(86) International application number: PCT/NZ2002/000132
(87) International publication number: WO 2003/009868

(56) References cited:
- WO-A-00/41682
- WO-A-01/75096
- WO-A1-91/05548
- WO-A1-93/16728
- WO-A1-94/16681
- CA-A1- 2 373 237
- US-A- 5 340 588
- GRIFFIN J.F.T ET AL.: 'Vaccine protocols to optimise the protective efficacy of BCG' TUBERCLE AND LUNG DISEASES vol. 79, no. 3, 1999, pages 135 - 143, XP002981159
- SAXEGAARD F. ET AL.: 'Control of paratuberculoses (Johne's disease) in goats by vaccination' THE VETERINARY RECORD vol. 116, no. 16, 20 April 1985, pages 439 - 441, XP002981149
- GRIFFIN J.F.T. ET AL.: 'BCG vaccination in deer: Distinctions between delayed type hypersensitivity and laboratory parameters of immunity' IMMUNOLOGY AND CELL BIOLOGY vol. 71, 1993, pages 559 - 570, XP002981146

## Description

### TECHNICAL FIELD

The present invention broadly relates to the use of lipids to formulate antigenic compositions, particularly live bacterial vaccines, and to methods for immunising animals using the compositions.

### BACKGROUND

Most human and animal pathogens including those that cause tuberculosis (TB), initiate infection via the mucosal surfaces. Accordingly, protective immunity against such pathogens may require induction of strong mucosal immune responses. However, mucosal immune responses are generally weak following parenteral immunisation. Despite the obvious need for vaccines, particularly TB vaccines, to protect against mucosal sites, the vaccines in use today are given by intradermal or subcutaneous injection. The development of more effective compositions, and/or delivery systems for vaccines by alternate routes is therefore desirable. Oral administration of vaccines in particular has a number of advantages including ease of administration and targeting of the mucosal immune response. Despite this, oral vaccination of animals and man to provide mucosal and/or systemic immunity has to date been largely ineffective. Efficacy of such vaccines has been hampered by degradation of the vaccine as it passes through the gut. In particular, most antigenic compounds possess peptide bonds that are readily broken down by gastric and proteolytic enzymes in the gut.

A number of vaccines rely on the use of freeze-dried preparations of organisms. For example, the current vaccine for human TB is based on freeze-dried preparations of a live attenuated bacterium called Bacille Calmette Guerin (BCG). However, it has been shown that freeze-drying procedures result in 30 to 50% loss of viability of BCG and impaired recovery of remaining live bacteria (7). A composition which retains greater viability of organisms prior to use would contribute greatly to the effectiveness of such vaccines.

To improve immune responses, antigens have been mixed with a number of adjuvant substances to stimulate immunogenicity. These adjuvants are primarily alum and oil-in-water emulsions. The latter group is typified by the Freund's mineral oil adjuvants. However, the use of Freund's complete adjuvant (FCA) in human and veterinary vaccines is contraindicated because of toxic reactions that have been reported. For these reasons, Freund's adjuvant may also be unsuitable for oral administration.
In other oil-in-water emulsions surfactants have been required because of the high oil content Detergent properties of surfactants render them unsuitable for parenteral or oral administration. Further, toxic reactions even for approved surfactants have been reported. A further drawback with emulsions are that they are heterogeneous systems of one immiscible liquid dispersed in another. This is unstable and results in separation of the aqueous phase over time, This poses difficulties for maintaining vaccines in stable suspension. Moreover, antigens trapped in the aqueous phase of water-in-oil emulsions are unlikely to be protected from degradation in the stomach.
liposomes and lipid vesicles have also been explored for use with vaccines, particularly with small antigenic components that may be readily encapsulated. Generally, liposomes and vesicles are not useful for encapsulation of large antigens such as live microorganisms. Moreover, liposomes and vesicles are costly and time consuming to produce, and the extraction procedures used in their preparation may result in alteration of the chemical structure or viability of vaccine preparations and hence their immunogenicity. For example, heat and solvents may alter the biological integrity of antigenic components such as proteins.

The patent WO 0041682 teaches a lipophilic solid microparticle having an average particle size ranging from 0.1 to 200 um, comprising a lipophilic substance and an active ingredient selected from the group consisting of a protein or peptide drug and an antigen. Used as oil dispersion O/W emulsion, the oral lipid formulations are suitable for stimulating mucosal immune response. The antigen may be obtained from 'live' viruses, Mycobacterium tuberculosis, anthrax, bacteria.

It is therefore an object of the present invention to provide an antigenic composition and/or delivery system which addresses these desiderata or which at least provides the public with a useful choice.

### SUMMARY OF THE INVENTION

Accordingly, in a first aspect the present invention provides an antigenic composition comprising a pharmaceutically acceptable lipid formulation and at least one antigenic component comprising an antigenically effective amount of live organisms, the composition being formulated for oral administration, as disclosed in the claims.

Preferred lipid formulations for use in the compositions of the invention contain long chain fatty acids.
In terms of fatty acid composition, a preferred lipid formulation contains 40% to 100%, preferably 60% to 100%, more preferably 80% to 100%, and even more preferably 90% to 100% C₁₆ and/or C₁₈ fatty acids.
A further preferred composition has a lipid formulation which contains less than 35%, preferably less than 25%, and more preferably less than 10% C₁₄ fatty acids or shorter.

In one embodiment, the lipid formulation contains:
20% to 60% saturated fatty acids;
25% to 60% monounsaturated fatty acids; and
0.5 to 15% polyunsaturated fatty acids.

In a particularly preferred composition, the lipid formulation contains:
35% to 50% saturated fatty acids;
40% to 55% monounsaturated fatty acids; and
5% to 9% polyunsaturated fatty acids.

The current preferred lipid formulation for use in the invention has the formula: 3% myristic acid; 26% palmitic acid; 15% stearic acid; 40% oleic acid; and 6% linoleic acid.
The antigenic component may be a protein, glycoprotein, peptide or factor with a protein or peptide component.

The antigenic component comprises live organisms. Preferably, as a biologically pure culture.
Preferably, the live organisms in the compositions of the invention are bacteria, particularly non-pathogenic bacteria, and more preferably bacteria belonging to the genus *Mycobacterium.*
A particularly preferred mycobacterium for use in the invention is *Mycobacterium bovis* BCG.

In one embodiment, the composition comprises at least two antigenic components. The first is preferably a live organism and the second antigenic component is preferably derived from an infectious agent, or is a weakly immunogenic protein or peptide.
In a further aspect, the invention provides a method for preparing an antigenic composition of the invention, the method comprising mixing the antigenic component(s) with the lipid formulation.
In a still further aspect, the invention also provides a method for immunising an animal, the method comprising administering to said animal an antigenic composition of the invention.
In a further aspect, the invention provides a method for stimulating a mucosal immune response in an animal, the method comprising administering to said animal an antigenic composition of the invention.
Administration of the composition in these methods is preferably by the oral route

### BRIEF DESCRIPTION OF THE DRAWINGS

Aspects of the invention will now be described in relation to the accompanying drawings in which:

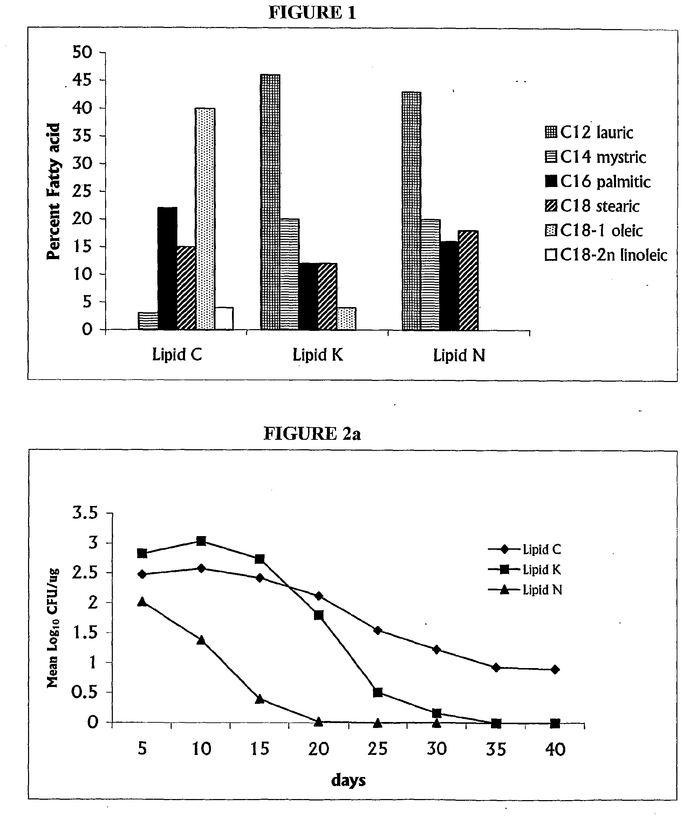

**Figure 1****.** Fatty acid composition of lipid formulations. Lipids were analysed by gas chromatography according to standard protocols. The fatty acid composition of each lipid is expressed as a percentage of the total fatty acid composition.

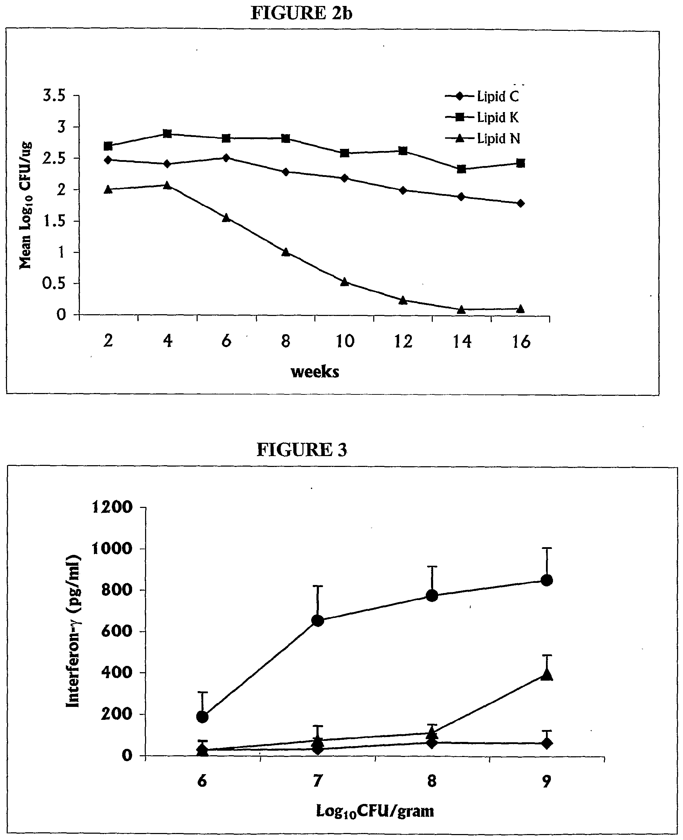

**Figure 2****.** BCG viability following formulation and storage in lipids at 4°C (2a) or room temperature (10-25°C) (2b). BCG formulations were warmed to 37°C and emulsified in 7H9 broth. Numbers of viable organisms were determined by inoculating serial 10 fold dilutions of each emulsion onto 7H11 agar plates. The number of CFU/ul of formulation media was determined after 2-3 weeks of culture. Results are representative of duplicate experiments and are expressed as means.
**Figure 3****.** Bovine PPD induced IFN-γ responses following oral vaccination with varying doses of formulated *M. bovis* BCG. Mice were sacrificed at 8 weeks after oral immunization with formulated *M. bovis* BCG (circles), non-formulated *M. bovis* BCG (triangles) or formulation material only (diamonds). Splenocytes were incubated with bovine PPD for 72 h. Supernatants were then collected and analysed using a sandwich ELISA. Each treatment group contained 6 mice. Spleens were individually processed. Results are expressed in pg/ml and are presented as means of triplicate determinations. Bar indicates standard error.

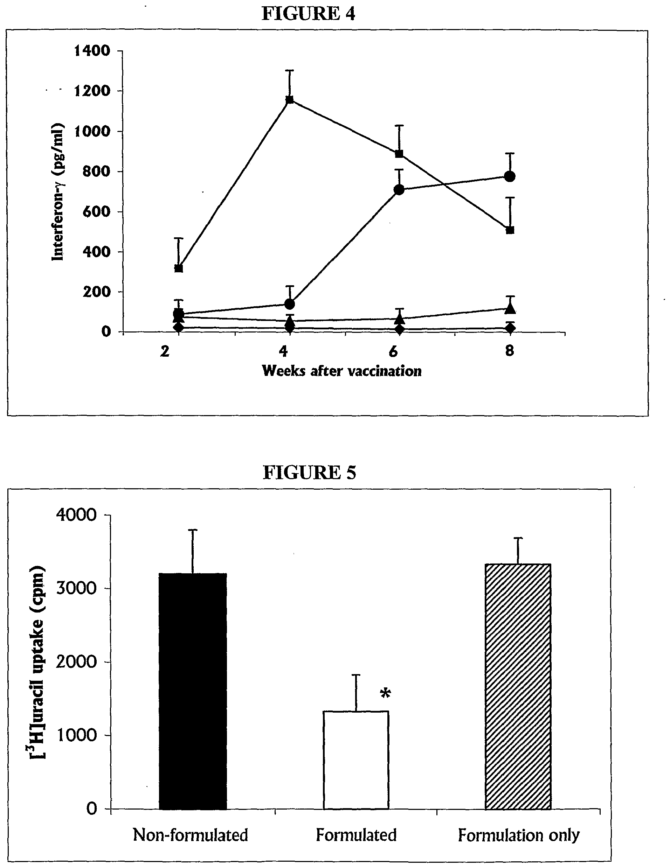

**Figure 4****.** Antigen-induced splenic IFN-γ responses to *M. bovis* BCG vaccination in BALB/c mice. Mice were euthanased at 2, 4, 6 and 8 weeks after vaccination with 10⁶ CFU subcutaneous *M. bovis* BCG (squares), oral delivery of 10⁷ CFU of formulated *M. bovis* BCG (circles), non-formulated *M. bovis* BCG (triangles), or formulation material only (diamonds). Splenocytes were incubated with bovine PPD for 72 h. Supernatants were then collected and analysed using a sandwich ELISA. Each treatment group contained 5-6 mice. Spleens were individually processed. Results are expressed in pg/ml and are presented as means of triplicate determinations. Results at 8 weeks are from 2 separate experiments. *P* value < 0.05 (Student *t* test). Bar indicates standard error.
**Figure 5****.** Growth inhibition of *M. bovis* by macrophages co-cultured with nonadherent peritoneal exudate cells (NPEC). Macrophages were infected with *M. bovis* at an MOI of 2 bacilli per macrophage. Non-adherent autologous NPEC were added at a ratio of 10 NPEC per macrophage. [³H]uracil incorporation was then assessed at 72h post infection. The mean [³H]uracil uptake by cell cultures which did not contain *M. bovis* was 460 cpm. Growth of intracellular bacilli from co-cultured macrophages and NPEC was expressed as means of triplicates. The results are representative of two experiments. *Represents a mean which is significantly different from the mean of Formulation only control group; bar indicates standard error.

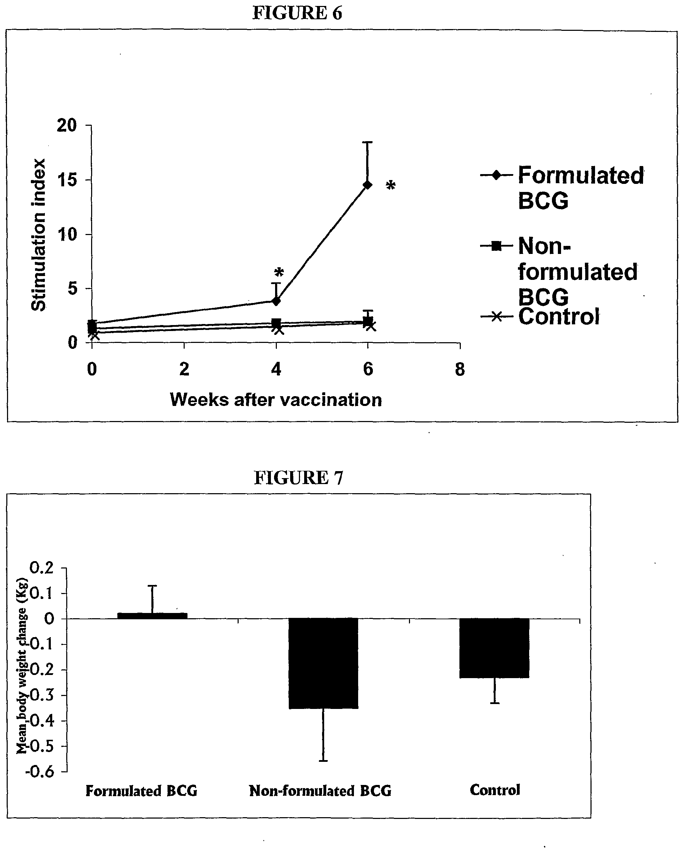

**Figure 6****.** Effect of oral vaccination of possums with formulated BCG on *in vitro* peripheral blood lymphocyte blastogenic responses to PPD-B. Formulated BCG ◆----◆; non-formulated BCG,■----■; non-vaccinated control **X**----**X**; Results are expressed as mean stimulation index (SI). *Represents a mean which is significantly different from the mean of non-vaccinated control group. Bar indicates SE.
**Figure 7****.** Effect of oral vaccination with formulated BCG on body weight of possums challenged with *M. bovis.* Mean body weight change was determined over the period from challenge to necropsy. The mean body weight of the possums immediately prior to challenge was 3.0 ± 0.07 (± SE) kg. Bar indicates SE.

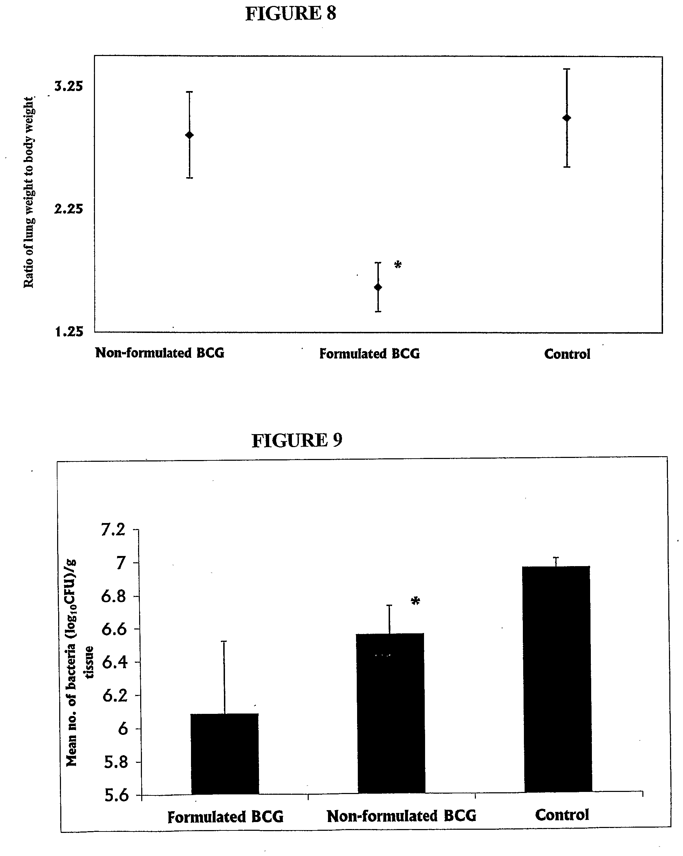

**Figure 8****.** Effect of oral vaccination with formulated BCG on lung weight of possums challenged with *M. bovis.* Mean lung weight was determined at necropsy. In order to standardise differences in lung weight with variation in body weight, the lung weight of each animal was compared with the body weight and expressed as a ratio. *Represents a mean which is significantly different from the mean of non-vaccinated control group; bar indicates SE.
**Figure 9****.** Effect of oral vaccination of possums with formulated BCG on mean numbers of mycobacteria isolated from lungs following challenge with *M. bovis.* Results are expressed as the geometric mean number of CFU (log₁₀)/g of tissue. *Represents a mean which is significantly different from the mean of non-vaccinated control group; bar indicates SE.

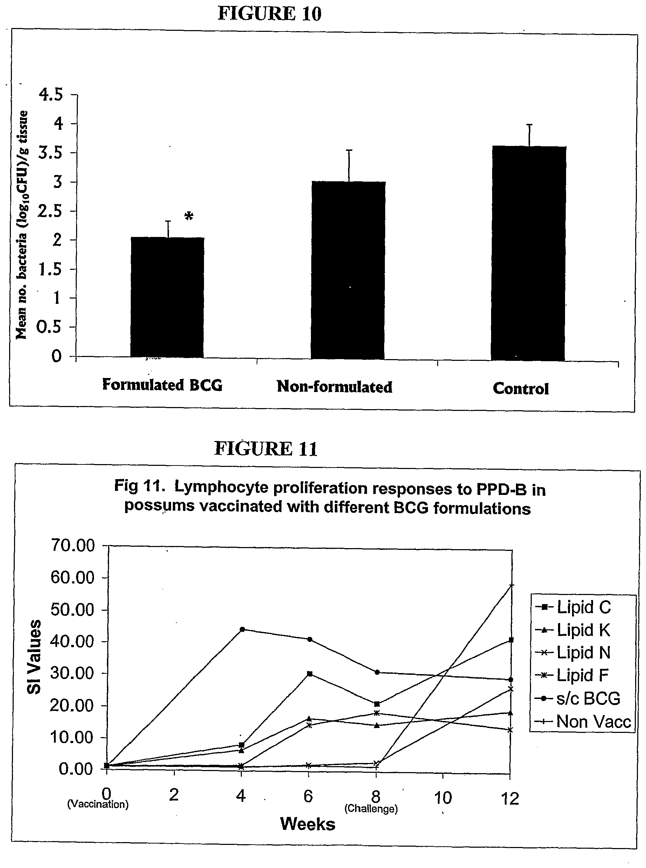

**Figure 10****.** Effect of oral vaccination of possums with formulated BCG on mean numbers of mycobacteria isolated from spleen following challenge with *M. bovis.* Results are expressed as the geometric mean number of CFU (log₁₀)/g of tissue. *Represents a mean which is significantly different from the mean of non-vaccinated control group; bar indicates SE.
**Figure 11****.** Comparison of immune responses to four oral lipid BCG formulations or to subcutaneous vaccination. The figure shows the effect on *in vitro* peripheral blood lymphocyte blastogenic responses to PPD-B in possums following vaccination (week 0) and challenge (week 8). Results are expressed as mean stimulation index (SI).

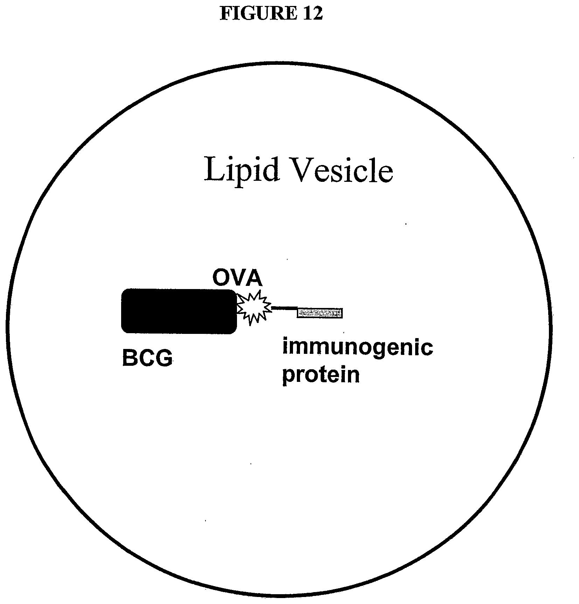

**Figure 12****.** is a diagram of a generic vaccine delivery system according to the invention.

### DETAILED DESCRIPTION

Accordingly, in a first aspect, the invention provides an antigenic composition comprising a pharmaceutically acceptable lipid formulation and at least one antigenic component comprising an antigenically effective amount of live organisms, the composition being formulated for oral administration.

Preferably, the lipid is in solid form. Conveniently, the lipid is in solid form at 10°C or above.

In a further aspect the present invention provides an antigenic composition comprising a pharmaceutically acceptable lipid formulation which is in solid form and undergoes solid to fluid transition between about 30°C to 40°C, and at least one antigenic component present in an antigenically effective amount

The lipids employed in the formulations above are preferably suitable for animal or human administration including consumption and may be selected from a broad range of natural (vegetable or animal derived), or synthetic lipid products including oils, fats and waxes.

Most usually, the lipid material will be liquid at temperatures above about 30°C. That is, the lipid should be selected to achieve melting point at physiological temperature in the animal to which it is administered, most usually by the oral route. Desirably, the lipid will be in the form of a solid at 10-30°C at atmospheric pressure, and preferably is still solid at from 20°C to 30°C at atmospheric pressure. However the melting temperature of lipid is not exclusive and may include oils, fats and waxes with a range of melting temperatures.

Preferred lipids for use herein will undergo transition from the solid phase to the liquid phase between about 30°C and physiological temperature of about 40°C, commonly 37°C. Summaries of lipid phase behaviour are available in the art, see for example (10). Accordingly, a skilled reader can select a lipid having the desired properties and melt point based on information in the art and simple experiment.

Suitable lipid formulations are triglycerides including glyceryl esters of carboxylic acids, compounds consisting of an aliphatic chain and a -COOH end, and saturated and non-saturated fatty acids and mixtures thereof.

Currently preferred lipids are triglycerides containing primarily C₈ to C₂₀ acyl groups, for example myristic, palmitic, stearic, oleic, linoleic, parinic, lauric, linolenic, arachidonic, and eicosapentaenoic acids, or mixtures thereof.

It has also been determined that for lipid formulations useful in the invention longer chain fatty acids, for example, C₁₆-C₁₈, are preferred. Long chain fatty acids have been found to be more effective in protecting organisms such as BCG in vaccines given to mice and possums. Viewed in this way, lipid formulations preferred for use in the invention contain: 40% to 100% , preferably 60% to 100%, preferably 80% to 100%, and more preferably 90% to 100% C₁₆ and/or C₁₈ fatty acids.

Generally, C₁₆ fatty acids represent from 10% to 40%, more preferably 20% to 35%, and even more preferably 25% to 32% of the total fatty acid content, and C₁₈ fatty acids represent from 40% to 90%, preferably from 50% to 80%, and more preferably from 60% to 70% C₁₈ of the total fatty acid content.

Preferred lipid formulations also contain less than 35% C₁₄ fatty acids or shorter, preferably less than 25%, and more preferably less than 10%.

In terms of chain length, the preferred lipid formulation contains less than 5% fatty acids with C₁₄ chains or shorter, 25% to 32% C₁₆ fatty acids, and from 60% to 70% C₁₈ fatty acid chains.

In terms of their fatty acid contents, lipid formulations for use in the invention may contain: saturated fatty acids in an amount from 20% to 60%, preferably 30% to 55%, and even more preferably 40% to 50%; monounsaturated fatty acids in an amount from 25% to 60%, preferably 30% to 60%, and more preferably 40% to 55%; and polyunsaturated fatty acids in an amount of from 0.5% to 15%, preferably 3% to 11%, and more preferably 5% to 9%.

A particularly preferred lipid formulation for use in the invention comprises 40% to 50% saturated fatty acids, 40% to 50% monounsaturated fatty acid, and 5% to 9% polyunsaturated fatty acid.

The currently preferred lipid formulation for use in the invention has the formula 3% myristic acid, 26% palmitic acid, 15% stearic acid, 40% oleic acid, and 6% linoleic acid as determined by HPLC analysis.

Currently preferred lipids formulations also include animal derived fractionated lipid complexes, one or more hydrogenated vegetable oils, especially olive oil or coconut oil, commercial suppository bases and other lipid formulations or mixtures thereof.

The lipid formulation is useful in the preparation of antigenic compositions, and in protecting antigens within the composition from degradation. The lipid formulation is especially useful in maintaining viability of live organisms, particularly bacteria. The lipid formulation acts to maintain the organisms in a live, but dormant state. This is particularly important for vaccines comprising live organisms formulated for oral administration. The lipids also maintain antigens in a uniform suspension. That is, in the compositions of the invention the antigenic components, and live organisms in particular, are uniformly distributed throughout a solid or paste like lipid matrix. The lipids also protect the antigens from destruction by gastrointestinal secretions when orally administered. Protection from macrophage attack is also likely when administered by other routes such as subcutaneously. This allows for uptake of the antigens and particularly live organisms through the gastrointestinal mucosa, and subsequent replication of organisms in the host. Replication of the live organisms within the host stimulates a protective immune response as determined by a reduction in severity of disease following challenge with virulent bacteria.

Formulations for a wide range of delivery routes may also include additives such as fillers, extenders, binders, wetting agents, emulsifiers, buffing agents, surfactants, suspension agents, preservatives, colourants, salts, antioxidants including mono sodium glutamate (MSG), vitamins such as vitamin E, butylated hydroxanisole (BHA), albumin dextrose-catalase (ADC), protective coatings, attractants and odourants, and agents to aid survival of organisms contained in the lipid but are not limited thereto.

Protective coatings or enterocoatings may be selected, for example, from gels, paraffins, and plastics including gelatin. The coatings further aid in the prevention of exposure to gastric acids and enzymes when the oral administration route is selected.

When used for oral administration, the formulation may also include additives which, for example, improve palatability, such as flavouring agents (including anise oil, chocolate and peppermint), and sweeteners (including glucose, fructose, or any other sugar or artificial sweetener).

The antigenic component may be a protein, glycoprotein, peptide, or factor with a protein or peptide component, preferably isolated and/or purified, or mixtures thereof. The component may be derived from an agent which may be used to generate an immune response in an animal.

Most usually, the antigen will bear at least one epitope which is present on an organism which is pathogenic in the animal species to be treated. Other antigenic structures such as are known in the art may also be used. For example, polysaccharides, glycolipids, and haptens conjugated to a carrier.

Preferably, the antigenic component is a living organism, preferably a biologically pure culture.

The living organism in the composition may be selected from the group consisting of: fungi, protozoans, bacteria and viruses. For example, HIV, SIV, Brucella and Anthrax. Preferably the organism is a bacterium. Organisms currently selected from non-pathogenic bacteria are preferred for use in compositions formulated for oral or subcutaneous delivery. A preferred bacterium is a non-pathogenic strain selected from the genus *Mycobacterium* including *M. tuberculosis* complex (comprising *M. tuberculosis, M. bovis, M. africanum* and *M. microtii*), *M. avium-intracellulare* complex (comprising *M. intracellulare* and *M. avium*), *M paratuberculosis, M. vaccae, M. smegmatis, M. chelonae, M. fortuitum, M. kansaii, M. leprae, M. marinum, M. ulcerans, M. simiae, M. haemophilum, M. malmoense, M. shimoidei, M. gastri, M. terrae complex,* and *M. nonchromogenicum.* In a particularly preferred embodiment the agent is *Bacille Calmette Guerin* (BCG), an attenuated strain of *M. bovis* including the following strains: 83/6235, Pasteur 1173P2, Glaxo 1077, Japanese 172, Prague, Russian, Brazilian, Danish 1331, Copenhagen, Connaught and including functionally equivalent variants and other attenuated strains of *M. bovis*, clones, mutants and recombinants of these strains either natural recombinants or those produced by any of a wide range of genetic engineering techniques, and antigenic components thereof.

It will be appreciated from the foregoing that the antigenic component may be a complex of proteins or peptides, or the like.

In one embodiment, the composition includes at least two antigenic components selected from any of those identified above, and may include multiple combinations of subunit antigens. Three or more antigenic components are feasible.

The concentration of the antigenic component(s) in the composition may vary according to known art protocols provided it is present in an amount which is effective to stimulate an immune response on administration to an animal. In particular, an immune response in the gut associated lymphoid tissue of the small intestine. In the case of mycobacteria a range of from 1 x 10⁵ to 1 x 10¹⁰ colony forming units (CFU)/ml is appropriate. Preferably, the concentration is from 1 x 10⁷ to 1 x 10⁹ CFU/ml. For protein and peptide type antigens a range of from 10-1000*µ*g per gram of formulation is appropriate. For virus-type antigens a range of 1 x 10³ to 1 x 10¹⁰, preferably 1 x 10⁵ to 1 x 10⁸ Plaque Forming Units (PFU)/ml is appropriate. The immune response may be humoral, or cell mediated including a mucosal immune response.

Accordingly, in a further aspect the invention relates to a method for stimulating a mucosal immune response in an animal by administering an antigenic composition of the invention to the animal.

The composition may be prepared using techniques known in the art. Conveniently, the lipid formulation is heated to liquefy if required, and the antigenic component(s) and other ingredients (when used) as described above are added. Dispersal of the antigenic composition may be achieved by mixing, shaking or other techniques that do not adversely affect the viability of the antigenic component.

Further preferred compositions for use in the invention are also essentially free of aqueous components including water. The term "essentially free" as used herein means that the composition contains less than 10% aqueous components, and preferably less than 5% aqueous components. As indicated above, the presence of components, particularly aqueous solvents, reduces the protective effect of the lipid formulation especially in the gut.

In one embodiment, the antigenic composition is a vaccine.

In an alternate embodiment, the antigenic composition is an adjuvant useful for administration with a vaccine to increase efficacy of same. Mycobacterium-containing, and BCG-containing antigenic compositions in particular are preferred for use as adjuvants.

The antigenic composition of the invention can also be useful for generating a response to a second or further antigenic molecule of a type as indicated above for the antigenic component, particularly those that are weakly immunogenic. This may be achieved by co-delivery of the second or further antigenic molecule in an antigenic composition of the invention by conjugating the antigenic molecule to the antigenic component of the composition. Conjugation may be achieved using standard art techniques (9). In particular, an antigen of interest may be conjugated to an antigenic carrier or adjuvant by a linker group which does not interfere with antibody production *in vivo.* The antigenic carrier or adjuvant may be any of the antigenic components including the organisms identified above but are preferably *Mycobacterium,* and more preferably BCG. Suitable linker groups include mannose receptor binding proteins such as ovalbumin and those that bind to Fc receptors. The second or further antigenic molecule is preferably a protein or peptide. A particularly preferred protein is an immunocontraceptive protein. The lipid again acts as the delivery matrix. An example of this vaccine delivery system is given in Figure 12. When the composition is administered an enhanced immune response to the conjugated molecule or co-delivered molecule results.

In a further aspect the invention also provides a method for immunising an animal, the method comprising administering to said animal an antigenic composition of the invention.

The term "animal" as used herein refers to a warm-blooded animal, and particularly mammals. Humans, dogs, cats, birds, cattle, sheep, deer, goats, rats, mice, rabbits, possums, badgers, guinea pigs, ferrets, pigs and buffalo are examples of animals within the scope of the meaning of the term. Monogastric and ruminant animals in particular are contemplated within this term.

The compositions of the invention may be administered by a variety of routes including parenteral (subcutaneous, intradermal, intramuscular), mucosal, aerosol and oral administration, but are not limited thereto. In one embodiment, oral administration is preferred. The compositions may be orally administered in the form of pellets, tablets, capsules, lozenges, or other suitable formulations. Oral administration enjoys wide consumer acceptance where the use of needles and syringes can be avoided and is an economical and practical method for vaccinating wildlife. In one embodiment the applicants have therefore provided a novel live vaccine formulated for oral administration.

In an alternate embodiment, the compositions may be formulated for parenteral administration by injection. This form of administration may also include injectable and subcutaneous depot formulations compatible with body tissues. Time release absorption from the depot may be achieved using the lipid formulation alone or with additional biodegradable polymers. The depot allows for sustained release of the antigenic component in a process which more closely approximates the infection process, facilitating the mounting of an immune response in the animal to which the composition is administered. A lipid protective effect also occurs with these forms of administration.

The composition can be administered as a single dose, particularly for parenteral administration, or in repeated doses over time. For example, an initial dose and booster doses at spaced intervals. The dosage for administration is determined by the release rate of the antigen component in combination with its antigenicity. Usual considerations such as weight, age, sex of the animal, concurrent treatments (if any), and nature of the antigen to be treated may also be taken into account. Generally the dose range for oral vaccination will be as given above, i.e. 1 x 10⁵ to 1 x 10¹⁰, preferably 1 x 10⁷ to 1 x 10⁹ CFU/kilogram per dose. For peptide and protein type antigens the dose range will be from 1-10,000*µ*g, preferably 10-1000*µ*g. For virus-type antigens the dose range will be from 1 x 10³ to 1 x 10¹⁰, preferably 1 x 10⁵ to 1 x 10⁸ PFU/ml. Whichever method of delivery is used, when live organisms are used in the vaccine formulation they are expected to multiply within the host to facilitate the immune response.

The composition may also be formulated as a single dose preparation or as a multidose preparation for mass vaccination programmes.

Until required for use, the compositions of the invention may be stored for limited periods at room temperature, or preferably under normal refrigeration conditions at approximately 4°C. At 4°C the lipid formulation facilitates storage and maintenance of organisms in a dormant but viable state without deterioration. For parenteral delivery, the composition is then warmed to 30 to 40°C to liquefy prior to administration. For oral administration the composition is a solid or a paste.

It will be appreciated that the above description is provided by way of example only and variations in both the materials and techniques used which are known to those persons skilled in the art are contemplated.

Non-limiting examples illustrating the invention will now be provided.

### EXAMPLES

### MATERIALS AND METHODS

**Bacteria.** *M. bovis* BCG Pasteur 1173P2 (Pasteur Institute, Paris) was used as the vaccine strain. The *M. bovis* strain used for macrophage infection studies and for possum challenge was *M. bovis* 83/6235 (AgResearch, Wallaceville, New Zealand) which was originally isolated from a tuberculous lesion in a brushtail possum and has been used in previous macrophage and possum inoculation studies (1, 4). For BCG formulation and macrophage infection, bacteria were grown to mid log phase in 175 ml flasks (Falcon) containing Middlebrook 7H9 medium (Difco, Detroit, Mich.) supplemented with albumin-dextrose-catalase (ADC; BBL, Becton Dickinson, Maryland, USA). Bacilli were harvested by centrifugation and washed twice in phosphate buffered saline (PBS) prior to storage at -70° C. For possum challenge, *M. bovis* was grown to mid-log phase in tween albumin broth (TAB) containing Dubos broth base (Difco Laboratories, Detroit, USA) supplemented with 0.006% v/v alkalinized oleic acid, 0.5% w/v albumin fraction V and 0.25% w/v glucose and the numbers of bacteria were estimated by the degree of turbidity. Dilutions for inoculating the possums were made in TAB. The number of colony forming units (CFU) of BCG or *M. bovis* was determined as described previously (5).

**Formulation composition.** Three lipid products were selected on the basis of melting temperature and the ability to maintain BCG in uniform suspension for formulating with BCG. Lipids which were liquid at 37°C but became solid below 30°C were chosen for testing in BCG viability studies. Following viability testing, the following three formulations were selected for testing in oral vaccine trials in mice and possums:
Formulation C -an animal derived fractionated complex lipid; Formulation K - consisting of tryglycerides of purified hydrogenated coconut oil; Formulation N-Novarta B, a commercially available suppository base. The three formulations were analysed by gas chromatography to determine the percentage of fatty acid groups.

**Formulation of BCG.** Pelleted BCG was resuspended in formulation medium which had been warmed to 37°C. BCG was resuspended at a concentration of 1 x 10⁷ CFU/ml for vaccination of mice or 1 x 10⁸ CFU/ml for vaccination of possums. To increase attractiveness and palatability for possums, 10 mg of glucose and 10 µl of anise oil (Pharmacare, Auckland NZ) were added per ml of formulation. For oral vaccination of mice, 10 mg of glucose, 1 mg of monosodium glutamate (Sigma), and 10% v/v ADC was added per ml of formulation. These additives were dispersed with the formulation lipids and were previously shown not to affect viability of BCG. BCG formulations were transferred to 15 ml tubes (falcon) and allowed to solidify with gentle mixing at 4°C. Formulations were removed from the tubes and aseptically cut into 1g pellets as required for viability testing and vaccination studies. Pellets were tested for dispersal of BCG by culturing on 7H11 agar plates and counting CFU as described below.

**BCG viability.** The number of CFU in the formulations following storage at 4°C or at room temperature (10-25°C) was determined as described previously (4). Samples for culture were collected by warming 100 mg aliquots of the three BCG formulations to 37°C for 15 min and performing serial 10-fold dilutions in 7H9 broth. Numbers of viable organisms were determined by inoculating 100 µl of each emulsion onto Middlebrook 7H11 agar plates (Difco) supplemented with oleic acid-ADC (OADC; Becton Dickinson) and 0.5 % glycerol. Emulsions were dispersed using a glass spreader. Plates were sealed with parafilm and incubated in 5% CO₂ at 37°C. The number of colonies was counted after 2-3 weeks of culture. Results are expressed as CFU/µg of BCG formulation.

**Vaccination of mice**. Specific pathogen free female BALB/c mice (6-8 weeks old) were obtained from the University of Otago Department of Animal Laboratory Sciences, Dunedin. Mouse experiments were conducted under ethics approval from the University of Otago Animal Ethics Committee (Approval No: 51/2000). Mice were separated into individual cages and taken off food for 12h prior to oral vaccination. Non-formulated controls consisted of *M. bovis* BCG in Craig's preservative-free strawberry jam (Heinz-Watties Ltd., Hastings, New Zealand). A previous study had shown that *M. bovis* BCG viability over a 24 h interval was not affected by mixing *M. bovis* BCG in the jam (data not shown). Non-vaccinated controls consisted of lipid formulation alone. For dose response and time course experiments mice were given vaccine in two separate doses at 24 h intervals. For aerosol challenge experiments, mice were given a single oral dose (5 x 10⁷ CFU) or vaccinated subcutaneously with 1 x 10⁶ CFU. The mice were observed at various intervals during consumption of pellets and jam to ensure the full dose was eaten. At various time-points post vaccination, the mice were sacrificed by CO₂ inhalation and their spleens were removed aseptically.

**Spleen cell proliferation assay.** Spleen cell suspensions were prepared by filtering cells through a cell strainer (70-µm mesh; Beckton Dickinson). Erythrocytes were lysed in 0.83% NH₄Cl (pH 7.2). Cells were washed twice in PBS and resuspended to 1 x 10⁶/ml in Dulbeccos's modified Eagles medium (DMEM) containing10% foetal calf serum (FCS), 20 mM HEPES penicillin at 100U/ml, streptomycin at 100µg/ml, 5.5 x 10⁻⁵ M 2-mercaptoethanol (DMEM-10%FCS; all from Gibco-BRL, USA). Cells were resuspended to a concentration of 10⁷ per ml in RPMI plus 10% foetal calf serum (Gibco). Splenocytes (5 x 10⁵ per well) were plated out in triplicate wells in 96-well plates (Nunc). Cells were cultured purified protein derivative from a culture of *M. bovis* (bovine PPD; CSL, Melbourne, Australia), 60µg/ml final concentration or with medium alone. Cells were harvested 4 days later, after an 18-h pulse with 1 µCi of [³H] thymidine (Amersham, Buckinghamshire, England)), and the incorporated thymidine was measured as previously described (5). A stimulation index (SI) was obtained by dividing the mean counts per minute (cpm) for the triplicate cultures incubated with bovine PPD by the mean cpm for splenocytes cultured with medium only.

***In vitro* assay for cytokine production by spleen cells.** Spleen cell suspensions were prepared as described above for the spleen cell proliferation assay. One ml of cell suspension was dispensed into 24 well plates (Costar) and 100 µl of either PBS or bovine PPD (60µg/ml final concentration) was added to the wells. Cultures were incubated for 72 h in 5% CO₂ at 37°C after which time 200 µl of culture supernatant was collected and frozen at 70°C for cytokine analysis. Interleukin-2 (IL-2) and interferon-gamma (IFN-γ) capture ELISAs were performed according to the manufacturers instructions using a commercial kit (R&D Systems, Duoset, City, Country) Cytokine levels in culture supernatants were quantified by extrapolation from standard curves. The minimum sensitivities of the two ELISAs were determined to be 50 pg/ml for IFN-γ and 35 pg/ml for IL-2.

***M. bovis* inhibition assay.** Peritoneal-derived macrophages were tested for inhibition of intracellular growth of *M. bovis* following co-culture with or without autologous lymphocytes. Experiments were performed according to a modification of previously described protocols. Peritoneal exudate cells (PEC) were obtained by lavage from female BALB/c mice. Cells were collected in PBS supplemented with 1% BSA and 20 U/ml of heparin, washed once and resuspended in DMEM medium containing 10% foetal calf serum and 100U/ml penicillin (supplemented DMEM) at 2 x 10⁶ /ml. 100 µl of cell suspension was dispensed into a 96 flat well plate (Nunc). After incubation for 2 h in 5% CO₂ at 37°C the nonadherent cells were removed, washed and resuspended at a density 5 x 10⁶/ml in supplemented DMEM. Nonadherent cells were selectively depleted of the remaining adherent population by incubation in 25 ml flasks (falcon). Nonadherent PEC (NPEC) were determined to comprise >90% lymphocytes following May-Grunwald/Giemsa staining. Warm supplemented DMEM was added to the adherent monolayer which was estimated to contain 5 x 10⁴ cells/well. This population was found to be 98% positive with a non-specific esterase staining kit (catalogue no. 181-B; Sigma, St. Louis, Mo, USA) and is henceforth referred to as macrophages. Macrophages were infected with *M. bovis* at an MOI of 2 bacilli per macrophage as described previously (2). Non phagocytosed bacteria were removed by gentle washing. One hundred µl (containing 5 x 10⁵ cells) of autologous NPEC was added to each well containing infected macrophages and cultures were further incubated in 5% CO₂ at 37°C. The resulting 10:1 NPEC-to-macrophage ratio was selected to approximate that of the ratio found in peripheral blood mononuclear cells. Control wells consisted of *M. bovis*-infected macrophages alone or uninfected NPEC and macrophages. After 72 h, cells were pulsed with 1.0 µCi [³H]uracil for 18 h. The cells were lysed with 0.1% saponin and the bacteria heat killed at 80-90°C for 20 minutes prior to harvesting onto glass fibre filters (Whatman Inc, Finland) using an automated cell harvester (Cambridge Technology, USA). The amount of [³H]uracil incorporated was determined using a liquid β-scintillation counter (Wallac, Country).

**Aerosol challenge of mice with** ***M. bovis**.* Six mice per vaccine group were challenged by aerosol with virulent *M. bovis* 8 weeks after vaccination. A single cell suspension of *M. bovis* 83/6235 was prepared using a modification of a method described by Grover et al., 1967 and stored at -70 C. For preparing these suspensions, the bacterial cells were dispersed by sonication for 30 seconds and filtered through an 8 µm membrane filter. Mice were infected via the respiratory route using an aerosol chamber which produces droplet nuclei of the size appropriate for entry into alveolar spaces. The concentration of viable *M. bovis* in the nebuliser fluid was empirically adjusted to result in the inhalation and retention of 5-20 viable organisms per mouse lungs (B. Buddle and G. de Lisle, unpublished data). A similar procedure has been shown to result in reproducible, uniform infection of the lungs of guinea pigs. The aerosol infection and subsequent maintenance and manipulation of infected mice were performed under strict isolation conditions in a biohazard facility.

**Isolation *of M. bovis.*** Mice were euthanased between 37 and 40 days after aerosol challenge. The lungs and spleen from each mouse were processed individually for mycobacterial isolation. The organs were homogenized in a Ten-Broeck grinder and samples centrifuged at 3500 g for 20 min. The deposits were resuspended in 1 ml of distilled water. Appropriate dilutions were made in TAB and a 0.1 ml volume of a diluted or undiluted sample was inoculated onto a modified mycobacteria 7H11 agar (1). Two replicates were prepared for each dilution. Culture conditions and methods for identification of isolates were carried out as previously described (1)

### Analysis of data

Statistically analyses of differences in the mean cytokine levels and log₁₀ transformed spleen cell proliferation responses for the vaccine groups were determined using the Student t test. The bacterial counts from the lung and spleen were log₁₀ transformed and analysed using analysis of variance. For statistical purposes, when no bacteria were cultured from tissues, half the lowest detectable count (% CFU/organ) was used.

**Vaccination and challenge of possums.** Possums were trapped and housed as previously described (4). BCG was fed to two groups of possums (5 animals/group). A 1 g pellet of formulated BCG (1 x 10⁸ CFU) was given to each possum in one group. A second group was given BCG (1 x 10⁸ CFU) in jam to control for the formulation procedure. The jam had previously been shown not to inhibit BCG viability (data not shown). A third group (6 animals/group) was given pellets containing formulation medium only and served as non-vaccinated controls. Possums were observed during consumption of pellets to ensure the full pellet was eaten. The following day the vaccinations were repeated (total BCG dose 2 x 10⁸ CFU/possum). All of the possums were challenged by the aerosol route 41 days after vaccination.

In a second experiment, four oral lipid BCG formulations were compared with subcutaneous vaccination. Six possums per vaccine group were challenged by aerosol with virulent *M. bovis* 8 weeks after vaccination. Lipid C,K.N and F (a modification of K containing 10% foetal calf serum)

**Aerosol challenge of possums with *M. bovis.*** The possums were challenged with *M. bovis* 83/6235, which was originally isolated from a lymph node of a possum from Taumaranui, New Zealand (5). Single cell suspensions of the isolate were prepared using a modification of a method described by Grover et al., 1967 and stored at -70°C. For preparing these suspensions, the bacterial cells were dispersed by sonication for 30 seconds and filtered through an 8 *µ* membrane filter. Possums anaesthetised with an intramuscular injection of ketamine HCl (30 mg/kg; Parnell Laboratories, Auckland, New Zealand) were infected via the respiratory route by using an aerosol chamber which produces droplet nuclei of the size appropriate for entry into alveolar spaces. The concentration of viable *M. bovis* in the nebuliser fluid was empirically adjusted to result in the inhalation and retention of 10-20 viable organisms per possum (Buddle and de Lisle, unpublished). This challenge dose had previously been estimated from the number of primary tubercles observed grossly in the lungs of non-vaccinated possums at 4 weeks post-infection. A similar procedure has been shown to result in reproducible, uniform infection of the lungs of guinea pigs (Wiegeshaus et al., 1970; Smith et al., 1970). The aerosol infection and subsequent maintenance and manipulation of infected possums were performed under strict isolation conditions in a biohazard facility.

**Necropsy of possums.** All possums were killed between 56 and 57 days after challenge and subjected to extensive gross post-mortem examination. The lungs were separated from surrounding tissues and weighed.

**Isolation of *M. bovis* from possum tissues.** From each animal, a sample of lung and spleen each weighing approximately 1 g, was taken from a macroscopic lesion, or, if no lesion was present, a sample was taken from a pre-determined part of the organ and processed individually for mycobacterial isolation. Samples were weighed, homogenized in a Ten-Broeck grinder and decontaminated in 0.75% cetyl-pyridinium chloride for 1 h. Samples were centrifuged at 3500 g for 20 min and deposits resuspended in 1 ml of distilled water. Appropriate dilutions were made in TAB and a 0.1 ml volume of a diluted or undiluted sample was inoculated onto a modified mycobacteria 7H11 agar plate. Two replicates were prepared for each dilution. Culture conditions and methods for identification of isolates were carried out as previously described (1).

**Possum peripheral blood lymphocyte proliferation assay.** Proliferative responses to PPD-B and PPD-A (CSL Limited, Parkville, Australia) were measured using whole blood depleted of red blood cells. Responses to Con A were also tested. Briefly, 1 ml of heparinised blood was mixed with 50 ml 0.17 M Tris- 0.16 M NH₄Cl, pH 7.2 at 37°C for 10 min, washed twice in PBS at 20°C and made up to 3 ml in DMEM tissue culture medium supplemented with 2mM glutamine and 2% normal possum serum. The cells (200 µl) were plated into flat bottom 96 well plates containing 50 µl PPD-B, PPD-A or Con A in PBS or PBS alone to give final concentrations of 60 µg/ml PPD or 5 µg/ml Con A. Plates were placed in a 5% CO₂ in air incubator for 72 hr, pulsed with 1µ Ci/well ³H-tritiated thymidine (Amersham, UK), harvested after a further 18 h and ³H counted in a Micro Beta Trilux (Wallac, Finland). The stimulation index (SI) was calculated by dividing counts per minute (cpm) from triplicate cultures stimulated with PPD by cpm from triplicate cultures with medium and PBS.

### Analysis of data

Statistically significant differences of mouse cytokine secretion were determined using the Student t test (GraphPad, San Diego, Calif.). These studies were performed twice with similar results. For possum lymphocyte proliferation responses, stimulation indices of >3.5 were scored as a positive response as this represents a response at least three standard deviations above the mean of the background (mean SI for PPD-B prior to vaccination). The possum body weight changes, lung weights, lymphocyte blastogenic responses and bacterial counts for the different treatment groups were initially compared by one-way analysis of variance. Duncan's multiple range test was then used to compare the means for individual groups. Lymphocyte proliferation responses and bacterial counts from the lung and spleen were log₁₀ transformed prior to analysis. For statistical purposes, when no bacteria were cultured from tissues, half the lowest detectable count (5 CFU/g tissue) was used.

### RESULTS

### A. Fatty acid composition of formulation lipids.

Lipids selected for use in formulating oral BCG were analysed by gas chromatography. Fig 1 shows the fatty acid composition of the 3 lipids used in mice and possum vaccination trials.

The relative percentage of fatty acids in the three lipid formulations are shown in Fig 1. Chemical analysis of lipids by HPLC showed that the 3 formulations comprised the following mixtures of fatty acids:

### Formulation C.

89% total lipid (48.5% neutral, 40.5% polar -comprising 3% myristic acid, 26% palmitic acid, 15% stearic acid, 40% oleic acid and 6% linoleic acid),

### Formulation K

47% lauric acid, 20% myristic acid, 12% palmitic acid, 12% stearic acid and 3% oleic acid.

Formulation N. Novarta B, a commercially available suppository base consisting of a mixture of esterified, hydrogenated, fractionated vegetable oils with synthetic triglyceride mixtures. comprising: 44% lauric acid, 20% myristic acid, 16% palmitic acid, 19% stearic acid.

### B. BCG viability following formulation.

The viability of formulated BCG following storage at 4°C is shown in Fig 2a. Over a period of 16 weeks, formulations C and K maintained high levels of BCG viability with formulation C showing higher retention of viability (98%) compared to formulation K (52%). In contrast, formulation N showed a progressive loss of BCG viability resulting in greater than 97% loss of viable organisms by 16 weeks. These results suggest that formulations C and K are more suited to maintaining BCG viability at 4°C compared to formulation N.

The viability of formulated BCG following storage at room temperature (10-25°C) is shown in Fig 2b. Formulations C and K maintained high levels of BCG viability with formulation C showing prolonged retention of viability (mean log₁₀ CFU/ug =10) at 40 days compared to formulation K (mean log₁₀ CFU/ug =10) at 22 days In contrast, formulation N showed a rapid loss of BCG viability (mean log₁₀ CFU/ug =10) at 12 days. These results suggest that formulations C and K are more suited to maintaining BCG viability at room temperature compared to formulation N.

### C. Immunogenicity of formulated BCG in mice.

**Oral delivery of formulated *M. bovis* BCG induces immune responses in mice.** To determine a suitable method of measuring systemic immune responses following oral delivery of *M. bovis* BCG, we compared bovine PPD-induced splenocyte proliferation (LTA), and splenic IL-2 and IFN-γ responses at 8 weeks following oral delivery of 10⁷ CFU of lipid-formulated *M. bovis* BCG or *M. bovis* BCG in jam (non-formulated *M. bovis* BCG). Table 1 shows that while both the LTA and IFN-γ assays showed significant differences between the formulated and non-formulated oral *M. bovis* BCG groups, the differences for the IL-2 assay were not significant. The IFN-γ assay was used in further experiments to monitor systemic immune responses due to importance of IFN-γ in protection against tuberculosis.

To determine the effect of dose of *M. bovis* BCG following oral delivery, we compared splenic IFN-γ responses to bovine PPD in mice vaccinated with varying doses of formulated or non-formulated *M. bovis* BCG at 8 weeks post vaccination. Fig 2 shows that a low level of IFN-γ (<200pg/ml) was detected in the formulated group following oral immunization with 10⁶ CFU of *M. bovis* BCG, but there were no significant differences between the vaccine groups. When the dose was increased to 10⁷ CFU, IFN-γ responses in the non-formulated group remained low whereas responses to formulated *M. bovis* BCG increased significantly (*P* < 0.05). Similar differences were seen with 10⁸ CFU of *M. bovis* BCG. When the vaccine dose was increased to 10⁹ CFU of BCG, an increase in the levels of IFN-γ was seen in the non-formulated group while the formulated group remained high. At doses of *M. bovis* BCG ranging from 10⁷-10⁹ CFU, IFN-γ responses in the formulated *M. bovis* BCG group were significantly greater that those of non-formulated *M. bovis* BCG. The increase in IFN-γ responses seen at the high dose in the non-formulated group shows that considerably higher doses of oral *M. bovis* BCG are required for induction of immune responses compared to formulated *M. bovis* BCG. To determine the time course of immune responses to oral *M. bovis* BCG, we compared splenic IFN-γ responses at 2 weekly intervals following oral or subcutaneous vaccination with *M. bovis* BCG. Fig. 3 shows that IFN-γ responses following subcutaneous vaccination peaked at 4 weeks and gradually declined at weeks 6 and 8. By comparison, IFN-γ responses following oral vaccination with formulated *M. bovis* BCG first increased at 6 weeks and remained high at 8 weeks post vaccination. IFN-γ responses to non-formulated *M. bovis* BCG or formulation material alone remained low between 2 and 8 weeks. These results show that immune responses following oral vaccination with formulated *M. bovis* BCG are delayed compared to subcutaneous vaccination but appear to persist at least to 8 weeks.

**Peritoneal-derived lymphocytes from mice orally vaccinated with formulated *M. bovis* inhibit growth *of M. bovis* in autologous macrophages.** The addition of NPEC to *M. bovis-*infected macrophages from mice vaccinated with oral *M. bovis* BCG formulations was performed in order to determine whether lymphocyte-mediated effector mechanisms could inhibit intracellular growth of *M. bovis.* Growth of *M. bovis* in macrophages was determined by [³H]uracil uptake. The growth of *M. bovis* within macrophages alone or when co-cultured with NPEC from orally vaccinated mice is illustrated in Fig. 4 Macrophages prepared from mice orally vaccinated with formulated or non-formulated *M. bovis* BCG or mice given formulation material alone showed no differences in their ability to control *M. bovis* growth. When NPEC from mice vaccinated with formulated *M. bovis* BCG were co-cultured with autologous *M. bovis*-infected macrophages, the [³H]uracil counts were significantly reduced compared to co-culture of NPEC from mice vaccinated with non-formulated *M. bovis* BCG or formulation material alone (*P*<0.05). These results demonstrate that lymphocytes from mice orally vaccinated with formulated *M. bovis* BCG activate macrophages to inhibit intracellular growth of *M. bovis.* Control of intracellular growth of *M. bovis* in vitro may reflect growth inhibition in vivo leading to reduced dissemination of *M. bovis* in the host.

**Oral vaccination with formulated *M. bovis* BCG protects against aerosol challenge with virulent *M. bovis.*** In order to determine the protective efficacy of formulated oral *M. bovis* BCG, mice were orally vaccinated with 5 x 10⁷ CFU formulated *M. bovis* BCG or subcutaneously vaccinated with 1 x 10⁶ CFU *M. bovis* BCG. Non-vaccinated mice served as controls. Mice were challenged with virulent *M. bovis* by the aerosol route 8 weeks after vaccination and euthanased 37-40 days after challenge. Table 2 shows that subcutaneous *M. bovis* BCG vaccination reduced the bacterial lung count by approximately 2.34 logs and the bacterial spleen count by 1.90 logs. By comparison, formulated oral *M. bovis* BCG reduced the bacterial lung count by approximately 1.0 log and the bacterial spleen count by 1.48 logs. The results in Table 2 showed that oral formulated *M. bovis* BCG and subcutaneous *M. bovis* BCG induced significant protection against aerosol challenge with virulent *M. bovis,* although the protective efficacy of subcutaneous *M. bovis* BCG in the lung was greater than that for oral formulated *M. bovis* BCG group.

### D. Immune responses and pathology in possums.

**Lymphocyte blastogenic responses.** The effect of oral vaccination with formulated BCG on the whole blood lymphocyte blastogenic responses to bovine PPD is shown in Fig.5 and Table 3. At 6 weeks after vaccination, the mean stimulation indices (SIs) to PPD-B for the formulated BCG group were significantly higher than for non-formulated BCG and non-vaccinated control groups (P<0.05). At 4 weeks following challenge with *M. bovis* all groups showed a mean SI for PPD-B of >20. These results show that oral delivery of formulated BCG elicits strong immune responses to PPD-B in possums compared to non-formulated BCG.

A further experiment compared immune responses to four oral lipid BCG formulations with subcutaneous vaccination (Fig 11), Subcutaneously vaccinated possums showed strong LTA responses which peaked at 4 weeks post vaccination (Mean SI 42.5) and gradually dropped to SI = 30 by 8 weeks In contrast, Lipid N formulated oral BCG failed to elicit an LTA response during the 8 week vaccination period. Oral BCG formulated in lipids C,K and F induced LTA responses which were weak (SI=1-7) at 4 weeks post-vaccination but increased progressively and were sustained through to 8 weeks post-vaccination (SI=15-22). These results show that systemic immune response to oral vaccination is delayed compared with subcutaneous vaccination but that they may persist longer. Formulation N did not induce LTA responses above those seen with the non-vaccinated possums nor did it protect against aerosol challenge with *M. bovis* (see table 4) indication that the type of lipid used to formulated oral BCG is important for protection against tuberculosis.

**Clinical findings.** The mean body weight changes between challenge and necropsy for the different groups are shown in Figure 6. The mean body weight of possums vaccinated with formulated BCG increased by 0.02 kg between the time of challenge and necropsy. In contrast, the mean body weights for the non-formulated BCG and non-vaccinated control groups decreased by 0.35 kg and 0.23 kg respectively during this period. However these differences were not statistically significant.

In a further experiment (table 4), which compared four oral lipid BCG formulations with subcutaneous vaccination, the mean body weight changes between challenge and necropsy were significantly reduced for the subcutaneous vaccination group (mean weight loss 0.012 kg) and one of the oral lipid BCG groups (group F) (0.035 kg) compared to the non-vaccinated group (0.147 kg). By comparison, the mean weight loss for the remaining oral BCG groups were 0,060 kg (lipid C), 0.067 kg (lipid K) and 0.122 kg (lipid N). Possums which did not show an immune response to vaccination (ie non-vaccinated and lipid N groups) showed greater body weight loss compared with those that responded.

**Pathology.** Macroscopic lesions were observed in the lungs of all of the challenged animals. The extent of tuberculous pneumonia can be estimated from the lung weights (Fig. 7). High lung weight is associated with extensive tuberculous pneumonia (3) (4). In order to standardise differences in lung weight with variation in body weight, the lung weight of each animal was compared with the body weight and expressed as a ratio. The ratio of mean lung weight to body weight of the animals vaccinated with formulated BCG was 1.62. By comparison the ratio of mean lung weight to body weight of the non-formulated BCG and non-vaccinated control groups were 2.86 and 3.0 respectively. The lung weight to body weight ratio of the possums vaccinated with formulated BCG was significantly different from the non-formulated BCG and non-vaccinated control groups (P<0.05). Typically, the lung lesions were small consolidated areas or lobar consolidation with a yellow necrotic area in the centre of the lesion. Swollen bronchial lymph nodes were observed in animals with the most extensive lung lesions.

In the second experiment (Table 4) which compared four oral lipid BCG formulations with subcutaneous vaccination, there were no significant differences in the ratio of mean lung weight to body weight between the vaccination groups. However possums which did not show an immune response to vaccination (ie non-vaccinated and lipid N groups) had higher mean lung weights compared with those that responded.

### Bacteriology

*Mycobacterium bovis* was isolated from the lung and spleens of the *M. bovis* challenged possums. The mean numbers of *M. bovis* isolated from the lungs and spleen for the different groups are shown in Figs 8 and 9. The mean lung bacterial counts for the non-formulated and formulated BCG groups were significantly lower than those for the non-vaccinated control group (P<0.05). The mean spleen bacterial counts for the formulated BCG group were approximately 10-fold less than the non-formulated BCG group and approximately 40-fold less than the non-vaccinated control group. The mean spleen bacterial counts for the formulated BCG group were significantly lower than those for non-formulated BCG and the non-vaccinated control groups (P<0.05).

In the second experiment which compared four oral lipid BCG formulations with subcutaneous vaccination (table 4), spleen bacterial counts for three of the orally vaccinated groups and the subcutaneously vaccinated group were significantly lower compared to the non-vaccinated group (P<0.05). The remaining oral lipid BCG group (lipid N) did not show significantly reduce bacterial spleen counts. No significant differences were seen between the groups when bacterial lung counts were compared. In general, possums which did not show an immune response to vaccination (ie non-vaccinated and lipid N groups) had higher mean bacterial spleen and lung counts compared with possums that had responded to vaccination.

**TABLE 1. Bovine PPD-stimulated spleen cell responses in mice 8 weeks after oral vaccination with different lipid formulations^{a}**

| Immunization | IL-2 (pg/ml) | IFN-γ (pg/ml) | LTA (SI) |
|---|---|---|---|
| Formulation only | 110.62 (+/-14.06) | 51.44 (+/-14.38) | 1.67 (+/-0.49) |
| Non formulated BCG | 153.51 (+/-25.22) | 65.10 (+/-20.05 | 1.83 (+/-0.51) |
| Formulation C BCG | 430.43* (+/-66.44) | 2160.95* (+/-273.40) | 16.26* (+/-1.20) |
| Formulation K BCG | 230.23 (+/-54.13) | 1268.30* (+/-76.80) | 7.26* (+/-0.83) |
| Formulation N BCG | 130.23 (+/-54.70) | 75.50 (+/-16.80) | 2.76 (+/-0.33) |

| | | | |
|---|---|---|---|
| *Represents a mean which is significantly different from the mean of non-vaccinated (Formulation only) control group. P value < 0.05 (Student *t* test) | | | |

**TABLE 2. Effect of vaccination on protection of mice against aerosol challenge with Mycobacterium bovis**

| Vaccine Group | Lung bacterial count^{†} | Log₁₀ resistance^{#} | Spleen bacterial count | Log₁₀ resistance^{#} |
|---|---|---|---|---|
| Non-vaccinated | 5.837^{a} (±0.362) | NA | 4.565^{a} (±0.189) | NA |
| Formulated Oral BCG | 4.774^{b} (±0.270) | 1.06 | 3.084^{b} (±0.176) | 1.48 |
| Subcutaneous BCG | 3.498^{c} (±0.237) | 2.34 | 2.660^{b} (±0.181) | 1.90 |

| | | | | |
|---|---|---|---|---|
| ^{†} Values are log₁₀ numbers of CFU ± standard error of *M. bovis* from the lungs and spleen of 6 animals per group 37-40 days post-challenge. ^{#} Data are expressed as levels of log₁₀ resistance calculated by subtracting the log₁₀ mean number of bacilli in the organs of vaccinated animals from the log₁₀ mean number of bacilli in the organs of non-vaccinated animals, NA-not applicable. Figures in columns with the same superscript letter are not significantly different (P>0.05). | | | | |

**TABLE 3. Number of possums responding to bovine PPD in the lymphocyte proliferation assay following oral vaccination.**

| Weeks after vaccination | | | | |
|---|---|---|---|---|
| BCG formulation | Group size | 0 | 4 | 6 |
| Formulated BCG | 5 | 0* | 2 | 5 |
| Non-formulated BCG | 5 | 0 | 0 | 1 |
| Control (no BCG) | 6 | 0 | 0 | 0 |

| | | | | |
|---|---|---|---|---|
| *No. of animals with stimulation index > 3.5 | | | | |

**TABLE 4. Pathological and microbiological findings for vaccinated possums challenged with M. bovis**

| **Vaccine Group** | **Change in body weight/challenge weight^{a}** | **Lung weight/PM body weight^{b}** | **Lung bacterial count^{c}** | **Spleen bacterial count^{c}** |
|---|---|---|---|---|
| | | | | |
| Lipid C | -0.060 | 22.54 | 5.634 | 1.301* |
| | (±0.033) | (±2.63) | (±0.385) | (±0.373) |
| | | | | |
| Lipid K | -0.067 | 15.49 | 5.481 | 1.321* |
| | (±0.030) | (±2.12) | (±0.428) | (±0.289) |
| | | | | |
| Lipid N | -0.122 | 23.47 | 6.038 | 2.200 |
| | (±0.037) | (±2.85) | (±0.273) | (±0.498) |
| | | | | |
| Lipid F | -0.035* | 16.28 | 5.342 | 0.934* |
| | (±0.031) | (±4.11) | (±0.290) | (±0.230) |
| | | | | |
| sc BCG | -0.012* | 20.07 | 5.384 | 1.270* |
| | (±0.055) | (±3.86) | (±0.427) | (±0.309) |
| | | | | |
| Non- | -0.147 | 24.10 | 6.048 | 2.553 |
| vaccinated | (±0.048) | (±3.50) | (±0.166) | (±0.465) |

| | | | | |
|---|---|---|---|---|
| Change in body weight /challenge body weight: Lipid F, BCG < Non-vaccinated (P<0.05). Spleen bacterial count: Lipid C, Lipid K, Lipid F, BCG < Non-vaccinated; Lipid F < Lipid N (P<0.05). ^{a} Change in body weight between post mortem and challenge (kg)/ body weight at challenge (kg) ^{b} Lung weight (g) / body weight at post mortem (kg). ^{c} Bacterial count, CFU log₁₀ / g of tissue. ^{*} Significantly different to non-vaccinated group. | | | | |

### INDUSTRIAL APPLICATION

The antigenic composition includes a lipid formulation which maintains antigens in a stable matrix, through which they are uniformly dispersed. This facilitates administration of consistent doses of antigen, avoiding dose dumping and ineffective low dosing. The lipid formulation has also been shown by the applicants to improve storage and viability of live organisms contained therein. The lipid formulation also protects the antigens and live organisms from degradation by stomach acids and enzymes. Losses in viability of organisms in lipid based formulations are also significantly lower than those reported for freeze-dried products. Storage under humid or moist conditions can also be achieved without deterioration because of the hydrophobic properties of the formulation.

It has been demonstrated that the viability of organisms, particularly bacteria in vaccine preparations is important for inducing strong and long lasting protective immunity. This may be achieved using the compositions of the invention. The compositions are also simple to prepare, more affordable to produce, and find increased consumer acceptance and safety where the use of needles and syringes can be avoided.

The inventive compositions may be administered in a variety of ways including subcutaneously, but are particularly amenable to oral delivery. The applicants have found that the lipid formulation in the composition can protect viability of organisms and their constituent antigens against degradation in the stomach, which enables live organisms to be taken up through the gastrointestinal mucosa for processing, replication and presentation to the immune system. Moreover, the applicants have determined that the doses to be administered can be effective at doses lower than previously anticipated for oral delivery (8).

Vaccination of wildlife, such as possums requires antigens to be delivered by the mucosal route. Oral bait vaccines therefore represent a practical and cost effective delivery option. Oral vaccination of humans is also a more cost effective method of vaccination and likely to find favour with users.

When administered in other ways such as subcutaneously, the lipid formulation still provides protection from attack, for example, by macrophages. With subcutaneous administration, or administration by injection, the formulation of a lipid depot also allows sustained release to mimic the infection process, and facilitate the mounting of an immune response.

It will be appreciated that the compositions of the invention also provide substantial advantages over many higher cost, injectable vaccine formulations.

The compositions are effective to induce immune responses to a wide range of infectious organisms, including gastrointestinal and respiratory pathogens, and preferably tuberculosis.

The compositions of the invention may also be used as a vaccine delivery system for a wide range of antigens, or for the co-delivery or conjugated delivery of antigenic molecules, particularly those which for reasons of dose or antigenicity are poorly immunogenic. The compositions of the invention are also useful as vaccine adjuvants.

It will further be appreciated by those persons skilled in the art that the present description is provided by way of example only and that the scope of the invention is not limited thereto.

### REFERENCE LISTING

1. Aldwell, F. E., D. L. Keen, V. C. Stent, A. Thomson, G. F. Yates, G. W. de Lisle, and B. M. Buddle. 1995. Route of BCG administration in possums affects protection against bovine tuberculosis. New Zealand Veterinary Journal. 43:356-359.
2. Aldwell, F. E., D. N. Wedlock, and B. M. Buddle. 1996. Bacterial metabolism, cytokine mRNA transcription and viability of bovine alveolar macrophages infected with Mycobacterium bovis BCG or virulent M. bovis. Immunol Cell Biol. 74:45-51.
3. Aldwell, F. E., D. N. Wedlock, and B. N. Buddle. 1997. Sequential activation of alveolar macrophages by IFN-γ and LPS is required for enhanced growth inhibition of virulent Mycobacterium bovis but not M.bovis BCG. Immunologiy and Cell Biology. 75**:.**
4. Buddle, B. M., F. E. Aldwell, D. L. Keen, N. A. Parlane, G. Yates, and G. W. de Lisle. 1997. Intraduodenal vaccination of brushtail possums with bacille Calmette-Guerin enhances immune responses and protection against Mycobacterium bovis infection. Int J Tuberc Lung Dis. 1:377-83.
5. Buddle, B. M., F. E. Aldwell, A. Pfeffer, and G. W. de Lisle. 1994. Experimental Mycobacterium bovis infection in the brushtail possum (Trichosurus vulpecula): pathology, haematology and lymphocyte stimulation responses. Vet Microbiol. 38:241-54.
6. Daugelat, S., C. H. Ladel, and S. H. Kaufmann. 1995. Influence of mouse strain and vaccine viability on T-cell responses induced by Mycobacterium bovis bacillus Calmette-Guerin. Infect Immun. 63:2033-40.
7. Gheorghiu, M., M. Lagranderie, and A. M. Balazuc. 1996. Stabilisation of BCG vaccines. New Approaches to Stablisation of Vaccine Potency. Dev.BIol.Stand.Basel, Karger. 87:251-261.
8. Lagranderie, M., P. Chavarot, A. M. Balazuc, and G. Marchal. 2000. Immunogenicity and protective capacity of Mycobacterium bovis BCG after oral or intragastric administration in mice. Vaccine. 18:1186-95.
9. Masarova, J.,Mislovicova, D.,Gemeiner, P and Michalkova, E. 2001 Stability enhancement of Escherichia coli penicillin G acylase by glycosylation with yeast mannan. Biotechnol Appl Biochem 34:127-33
10. Morrison, I. M. and Hawke, J. C 1979. Influence of elevated levels of linoleic acid on the thermal properties of bovine milk fat Lipids 14: 391-4.

## Claims

1. An antigenic composition for oral administration comprising a lipid formulation and at least one antigenic component comprising an antigenically effective amount of live organisms; wherein the lipid formulation is in paste or solid form and is solid at a temperature of 10°C or above, wherein the lipid formulation undergoes solid to fluid transition between about 30°C to 37°C and contains:
(a) 40% to 100%, preferably 60% to 100%, preferably 80% to 100%, and more preferably 90% to 100% C₁₆ and/or C₁₈ fatty acids; or
(b) contains 10% to 40%, preferably 20% to 35%, and more preferably 25% to 32% C₁₆ fatty acids; and 40% to 90%, preferably 50% to 80%, and more preferably 60% to 70% C₁₈ fatty acids; or
(c) contains less than 35%, preferably less than 25%, and more preferably less than 10% C₁₄ fatty acids or shorter; or
(d) contains less than 5% C₁₄ fatty acids or shorter, 25% to 32% C₁₆ fatty acids; and 60% to 70% C₁₈ fatty acids.

2. An antigenic composition for oral administration comprising a lipid formulation and at least one antigenic component comprising an antigenically effective amount of live organisms; wherein the lipid formulation is in paste or solid form and is solid at a temperature of 10°C or above, wherein the lipid formulation undergoes solid to fluid transition between about 30°C to 37°C and contains:
20% to 60% saturated fatty acids,
25% to 60% monounsaturated fatty acids; and
0.5% to 15% polyunsaturated fatty acids

3. A composition according to claim 2, wherein the lipid formulation contains:
40% to 50% saturated fatty acids;
40% to 55% monounsaturated fatty acids; and
5% to 9% polyunsaturated fatty acids.

4. A composition according to claim2 or 3, wherein the lipid formulation has the formula: 3% myristic acid; 26% palmitic acid; 15% stearic acid; 40% oleic acid; and 6% linoleic acid.

5. A composition according to any preceding claim, further comprising another antigen selected from the group of proteins, glycoproteins, peptides, or factors with a protein or peptide component, or mixtures thereof.

6. A composition according to claim 5, wherein the protein is an immunocontraceptive protein.

7. A composition according to claim 5 or claim 6, wherein the protein or peptide is poorly immunogenic.

8. A composition according to any preceding claim, wherein the live organisms are selected from the group consisting of fungi, protozoa, bacteria, and viruses.

9. A composition according to claim 8, wherein the virus is HIV or SIV.

10. A composition according to claim 8, wherein the bacteria are selected from Brucella, Anthrax and *Mycobacterium.*

11. A composition according to claim 10, wherein the bacterium is *Mycobacterium.*

12. A composition according to claim 12, wherein the *Mycobacterium* is selected from *M*. *tuberculosis* complex (comprising *M. tuberculosis*, *M. bovis*, *M. africanum* and *M*. *microtii)*, *M. avium-intracellulare* complex (comprising *M. intracellulare* and *M. avium), M. paratuberculosis, M. vaccae, M. smegmatis, M. chelonae, M. fortuitum*, *M. kansaii*, *M. leprae, M. marinum*, *M*. *ulcerans*, *M. simiae*, *M. haemophilum*, *M. malmoense*, *M. shimoidei*, *M. gastri*, *M. terrae* complex, and *M*. *nonchromogenicum*.

13. A composition according to claim 11, wherein the *Mycobacterium* is *M. bovis.*

14. A composition according to claim 12 or claim 13, wherein the *M. bovis* is Bacille Calmette Geurin (BCG).

15. A composition according to claim 14, wherein the BCG is a strain selected from 83/6235, Pasteur 1173P3, Glaxo 1077, Japanese 172, Prague, Russian, Brazilian, Danish 1331, Copenhagen and Connaught.

16. A composition according to any preceding claim, wherein the lipid formulation is solid at 10°C to 30°C.

17. A composition according to claim 16, wherein the lipid formulation is solid at 20°C to 30°C.

18. A composition according to any preceding claim which is essentially free of aqueous components.

19. A composition according to any preceding claim, wherein the lipid formulation contains coconut oil, hydrogenated coconut oil, olive oil, hydrogenated olive oil, an attractant, an odourant or a flavouring agent.

20. A composition according to any preceding claim for use in immunising an animal or stimulating mucosal immune response in an animal.

## Patentansprüche

1. Eine antigenische Zusammensetzung zur oralen Administration, umfassend eine Lipidformulierung und mindestens eine antigenische Komponente, umfassend eine antigenisch effektive Menge an lebenden Organismen, wobei die Lipidformulierung in Form einer Paste oder in fester Form ist oder bei einer Temperatur von 10°C oder darüber fest ist, wobei die Lipidformulierung zwischen etwa 30°C bis 37°C einen fest-zuflüssig Übergang durchläuft und enthält:
(a) 40% bis 100%, bevorzugt 60% bis 100%, bevorzugt 80% bis 100% und bevorzugter 90% bis 100% C₁₆ und/ oder C₁₈ Fettsäuren oder
(b) 10% bis 40%, bevorzugt 20% bis 35% und bevorzugter 25% bis 32% C₁₆ Fettsäuren enthält und 40% bis 90%, bevorzugt 50% bis 80% und bevorzugter 60% bis 70% C₁₈ Fettsäuren oder
(c) weniger als 35%, bevorzugt weniger als 25% und bevorzugter weniger als 10% C₁₄ Fettsäuren oder kürzer enthält oder
(d) weniger als 5% C₁₄ Fettsäuren oder kürzer, 25% bis 32% C₁₆ Fettsäuren und 60% bis 70% C₁₈ Fettsäuren enthält.

2. Eine antigenische Zusammensetzung zur oralen Administration, umfassend eine Lipidformulierung und mindestens eine antigenische Komponente, umfassend eine antigenisch effektive Menge an lebenden Organismen, wobei die Lipidformulierung in Form einer Paste oder in fester Form ist oder bei einer Temperatur von 10°C oder darüber fest ist, wobei die Lipidformulierung zwischen etwa 30°C bis 37°C einen fest-zuflüssig Übergang durchläuft und enthält:
20% bis 60% gesättigte Fettsäuren,
25% bis 60% einfach ungesättigte Fettsäuren und
0,5% bis 15% mehrfach ungesättigte Fettsäuren.

3. Eine Zusammensetzung nach Anspruch 2, wobei die Lipidformulierung enthält:
40% bis 50% gesättigte Fettsäuren;
40% bis 55% einfach ungesättigte Fettsäuren; und
5% bis 9% mehrfach ungesättigte Fettsäuren.

4. Eine Zusammensetzung nach Anspruch 2 oder 3, wobei die Lipidformulierung die Formel hat: 3% Myristinsäure, 26% Palminsäure; 15% Stearinsäure; 40% Ölsäure und 6% Linolsäure.

5. Eine Zusammensetzung nach einem der vorhergehenden Ansprüche, ferner umfassend ein anderes Antigen ausgewählt aus der Gruppe aus Proteine, Glykoproteine, Peptide oder Faktoren mit einer Protein- oder Peptidkomponente oder Mischungen davon.

6. Eine Zusammensetzung nach Anspruch 5, wobei das Protein ein immunkontrazeptives Protein ist.

7. Eine Zusammensetzung nach Anspruch 5 oder Anspruch 6, wobei das Protein oder Peptid schwach immunogen ist.

8. Eine Zusammensetzung nach einem der vorhergehenden Ansprüche, wobei die lebenden Organismen ausgewählt sind aus der Gruppe bestehend aus Pilzen, Protozoen, Bakterien und Viren.

9. Eine Zusammensetzung nach Anspruch 8, wobei das Virus HIV oder SIV ist.

10. Eine Zusammensetzung nach Anspruch 8, wobei die Bakterien ausgewählt sind aus Brucella, Anthrax und *Mycobacterium.*

11. Eine Zusammensetzung nach Anspruch 10, wobei das Bakterium *Mycobacterium* ist.

12. Eine Zusammensetzung nach Anspruch 12, wobei das *Mycobacterium* ausgewählt ist aus *M*. *tuberculosis* complex (umfassend *M*. *tuberculosis, M. bovis, M. africanum* und *M*. *microtii*)*, M. avium-intracellulare* Komplex (umfassend *M*. *intracellulare* und *M*. *avium*), *M. paratuberculosis, M. vaccae, M. smegmatis, M. chelonae, M. fortuitum, M. kansaii, M. leprae, M. marinum, M. ulcerans, M. simiae, M haemophilum, M malmoense, M shimoidei, M gastri, M terrae* Komplex, und *M. nonchromogenicum.*

13. Eine Zusammensetzung nach Anspruch 11, wobei das *Mycobacterium M. bovis* ist.

14. Eine Zusammensetzung nach Anspruch 12 oder Anspruch 13, wobei das *M*. *bovis* Bacille Calmette Geurin (BCG) ist.

15. Eine Zusammensetzung nach Anspruch 14, wobei das BCG ein Stamm ist ausgewählt aus 83/6235, Pasteur 1173P3, Glaxo 1077, Japanese 172, Prague, Russian, Brazilian, Danish 1331, Copenhagen und Connaught.

16. Eine Zusammensetzung nach einem der vorhergehenden Ansprüche, wobei die Lipidformulierung bei 10°C bis 30°C fest ist.

17. Eine Zusammensetzung nach Anspruch 16, wobei die Lipidformulierung bei 20°C bis 30°C fast ist.

18. Eine Zusammensetzung nach einem der vorhergehenden Ansprüche, die essentiell frei von wässrigen Komponenten ist.

19. Eine Zusammensetzung nach einem der vorhergehenden Ansprüche, wobei die Lipidformulierung Kokosnussöl, hydriertes Kokosnussöl, Olivenöl, hydriertes Olivenöl, einen Lockstoff, einen Duftstoff oder einen Aromastoff enthält.

20. Eine Zusammensetzung nach einem der vorhergehenden Ansprüche zur Verwendung bei der Immunisierung eines Tieres oder der Stimulation der mukosalen Immunantwort in einem Tier.

## Revendications

1. Composition antigénique pour une administration orale, comprenant une formulation lipidique et au moins un composant antigénique comprenant une quantité antigéniquement efficace d'organismes vivants, la formulation lipidique étant sous une forme de pâte ou sous une forme solide et étant solide à une température de 10°C ou au-dessus, la formulation lipidique subissant une transition solide à fluide entre environ 30°C et 37°C et contenant :
(a) 40 % à 100 %, de préférence 60 % à 100 %, de préférence 80 % à 100 %, et de façon davantage préférée 90 % à 100 % d'acides gras en C₁₆ et/ou C₁₈ ; ou
(b) contenant 10 % à 40 %, de préférence 20 % à 35 %, et de façon davantage préférée 25 % à 32 % d'acides gras en C₁₆ ; et 40 % à 90 %, de préférence 50 % à 80 %, et de façon davantage préférée 60 % à 70 % d'acides gras en C₁₈ ;
ou
(c) contenant moins de 35 %, de préférence moins de 25 %, et de façon davantage préférée moins 10 % d'acides gras en C₁₄ ou plus courts ; ou
(d) contenant moins de 5 % d'acides gras en C₁₄ ou plus courts, 25 % à 32 % d'acides gras en C₁₆ ; et 60 % à 70 % d'acides gras en C₁₈.

2. Composition antigénique pour une administration orale, comprenant une formulation lipidique et au moins un composant antigénique comprenant une quantité antigéniquement efficace d'organismes vivants, la formulation lipidique étant sous une forme de pâte ou sous une forme solide et étant solide à une température de 10°C ou au-dessus, la formulation lipidique subissant une transition solide à fluide entre environ 30°C et 37°C et contenant :
20 % à 60 % d'acides gras saturés,
25 % à 60 % d'acides gras mono-insaturés ; et
0,5 % à 15 % d'acides gras polyinsaturés.

3. Composition selon la revendication 2, dans laquelle la formulation lipidique contient :
40 % à 50 % d'acides gras saturés ;
40 % à 55 % d'acides gras mono-insaturés ; et
5 % à 9 % d'acides gras polyinsaturés.

4. Composition selon l'une des revendications 2 ou 3, dans laquelle la formulation lipidique a la formule : 3 % d'acide myristique ; 26 % d'acide palmitique ; 15 % d'acide stéarique ; 40 % d'acide oléique ; et 6 % d'acide linoléique.

5. Composition selon l'une quelconque des revendications précédentes, comprenant en outre un autre antigène choisi dans le groupe consistant en protéines, glycoprotéines, peptides, ou facteurs avec un composant protéique ou peptidique, ou les mélanges de ceux-ci.

6. Composition selon la revendication 5, dans laquelle la protéine est une protéine immunocontraceptive.

7. Composition selon la revendication 5 ou la revendication 6, dans laquelle la protéine ou le peptide est médiocrement immunogénique.

8. Composition selon l'une quelconque des revendications précédentes, dans laquelle les organismes vivants sont choisis dans le groupe consistant en champignons, protozoaires, bactéries et virus.

9. Composition selon la revendication 8, dans laquelle le virus est le VIH ou le VIS.

10. Composition selon la revendication 8, dans laquelle les bactéries sont choisies parmi Brucella, Anthrax et *Mycobacterium.*

11. Composition selon la revendication 10, dans laquelle la bactérie est *Mycobacterium.*

12. Composition selon la revendication 12, dans laquelle le *Mycobacterium* est choisi parmi le complexe de *M. tuberculosis* (comprenant *M. tuberculosis, M. bovis, M. africanum* et *M. microtii*), le complexe de *M. avium-intracellulare* (comprenant *M*. *intracellulare* et *M avium*), *M. paratuberculosis, M. vaccae, M. smegmatis, M. chelonae, M. fortuitum, M. kansaii, M. leprae, M. marinum, M. ulcerans, M. simiae, M. haemophilum, M. malmoense, M. shimoidei, M. gastri,* le complexe de *M*. *terrae,* et *M*. *nonchromogenicum.*

13. Composition selon la revendication 11, dans laquelle le *Mycobacterium* est *M*. *bovis.*

14. Composition selon la revendication 12 ou la revendication 13, dans laquelle le *M*. *bovis* est le Bacille de Calmette et Guérin (BCG).

15. Composition selon la revendication 14, dans laquelle le BCG est une souche choisie parmi 83/6235, Pasteur 1173P3, Glaxo 1077, Japanese 172, Prague, Russian, Brazilian, Danish 1331, Copenhagen et Connaught.
3

16. Composition selon l'une quelconque des revendications précédentes, dans laquelle la formulation lipidique est solide à 10°C à 30°C.

17. Composition selon la revendication 16, dans laquelle la formulation lipidique est solide à 20°C à 30°C.

18. Composition selon l'une quelconque des revendications précédentes, qui est sensiblement exempte de composants aqueux.

19. Composition selon l'une quelconque des revendications précédentes, dans laquelle la formulation lipidique contient de l'huile de noix de coco, de l'huile de noix de coco hydrogénée, de l'huile d'olive, de l'huile d'olive hydrogénée, un agent d'attraction, une substance odorante ou un agent aromatisant.

20. Composition selon l'une quelconque des revendications précédentes, pour une utilisation dans l'immunisation d'un animal ou la stimulation d'une réponse immunitaire mucosale chez un animal.
